# EUROPEAN PATENT SPECIFICATION

(11) **EP 1 046 400 B1**
(45) Date of publication and mention of the grant of the patent: **22.06.2005**
(21) Application number: 99400964.5
(22) Date of filing: 20.04.1999
(51) Int. Cl.: A61K 38/45, A61K 48/00, A61P 35/00

(54) **Use of an Equine herpes virus type-4 thymidine kinase and a nucleoside analog selected from ganciclovir and acyclovir for the killing of human proliferative cells**
Verwendung von equiner-Herpes-Virus-typ-4-Thymidin-Kinase und einen Nucleosidanalog ausgewählt von Ganciclovir und Acyclovir zur Tötung von proliferativen Humanzellen
Utilisation d'une tymidine kinase d'herpès equin de type 4 et d'un analoge de nucleoside choisi parmi le ganciclovir et l'acyclovir pour tuer des cellules prolferantes humaines

(43) Date of publication of application: 25.10.2000
(73) Proprietor: Université Pierre et Marie Curie (Paris VI), 75252 Paris Cédex 05 (FR)
(72) Inventor: Klatzmann, David, 75013 Paris (FR); Loubiere, Laurence, 75012 Paris (FR)
(74) Representative: Becker, Philippe

(56) References cited:
- WO-A-92/01045
- WO-A-95/22617
- ZHAO Y ET AL: "Regulatory function of the equine herpesvirus 1 ICP27 gene product." JOURNAL OF VIROLOGY, (1995 MAY) 69 (5) 2786-93. , XP002126519

## Description

The present invention relates to the killing of human proliferative cells in vitro, ex vivo or in vivo. More specifically, this invention provides improved means for rendering human cells sensitive to nucleoside analogs. The invention relies in particular on the discovery of advantageous properties of certain polypeptides and nucleic acids as conditional suicide agents, more particularly of the thymidine kinase type. The invention can be used in various technical areas such as experimental biology, therapy, prophylaxy, screening methods, etc.

Mammalian cells that express a conditional suicide gene are selectively killed or destroyed by activation of a prodrug into a toxic metabolite. The Herpes Simplex virus type 1 thymidine kinase gene (HSV1-TK) is the most widely used suicide gene, both in experimental settings and clinical trials.¹ Expression of the HSV1-TK gene renders tumor cells sensitive to antiviral agents like acyclovir (ACV), ganciclovir (GCV) and bromovinyl-deoxyuridine (BVDU). These nucleoside analogs are efficiently converted to their monophosphate form by HSV1-TK, and are then converted to triphosphate compounds by host cellular kinases.²⁻⁴ Incorporation of these metabolites into elongating DNA blocks elongation leading to cell death.⁵⁻⁸ The HSV1-TK/GCV system has proved efficient for inducing the regression of transplanted tumors in various animal models,⁹⁻¹¹ as well as of carcinogen induced tumors.¹² Based on these favorable results, several clinical gene therapy trials are in progress aimed to assess safety and efficacy of this treatment for treating malignancies¹³ or other proliferative diseases such as Graft versus Host diseases, and preliminary results have now been reported.¹⁴⁻¹⁷

Despite the effectiveness of the HSV1-TK/GCV system for killing proliferating cells (e.g., tumor cells), it remains important to try to improve it, with the aim to maximize therapeutic efficacy and/or to facilitate treatment modalities. For example, GCV has the disadvantage of being administered by slow intra-venous perfusion, requiring patient hospitalization. It would thus be interesting to find nucleoside analogs with an oral administration having the same efficacy as the GCV prodrug. It would also represent a significant achievement to find ways to improve the efficacy of the killing effect observed with a TK. In this regard, it has been proposed in the art to use stronger promoter regions to increase the cellular levels of thymidine kinase. It has also been suggested to use a combination of several enzymes in order to increase the phosphorylation efficacy. It has furthermore been proposed to produce and screen for mutants or other derivatives of HSV-1 TK, with improved affinity or activity towards nucleoside analogs.

The present invention now provides alternative means which address the above problems and, more particularly, which provide for an improved sensitivity of human cells to nucleoside analogs. More specifically, the Applicants have now unexpectedly shown that an increased sensitivity to nucleoside analogs selected from Ganciclovir or acyclovir can be obtained in human cells with a particular type of thymidine kinase, i.e., an Equine herpesvirus type-4 thymidine kinase polypeptide or nucleic acid. In particular, by using wild-type Equine herpesvirus type-4 thymidine kinase (wtEHV4-TK) or variants thereof, the Applicants have demonstrated that human cells become 3 to 12 fold more sensitive to ganciclovir or acyclovir than with HSV-1 TK. The results obtained show that EHV4-TK provides a very interesting alternative suicide gene to the HSV1-TK gene for many uses, including experimental, biological, screening or therapeutical applications.

One object of the present invention therefore resides in the use of an Equine herpes virus type-4 (EHV4) thymidine kinase polypeptide or a nucleic acid coding therefor for the manufacture of a composition which renders human cells sensitive to a nucleoside analog selected from ganciclovir or acyclovir in vitro, ex vivo or in vivo.

Within the context of the present invention, the term Equine herpes virus type-4 thymidine kinase polypeptide ("EHV4-TK") designates either wild-type EHV4-TK or any biologically active fragment or variant thereof. The wild type EHV4-TK has been isolated, characterized and its amino acid sequence has been published in the art.¹⁸ This polypeptide can be obtained by any conventional method known in the art such as recombinant production, artificial synthesis or purification from EHV4-infected cells. A biologically active fragment or variant of wild-type EHV4-TK means inter alia a polypeptide having the ability to phosphorylate a nucleoside analog as defined below such as, for instance, GCV, ACV or BVDU.

Fragments of EHV4-TK can be prepared by any conventional method such as chemical or enzymatic cleavage followed by separation of the fragments, recombinant production from a truncated nucleic acid, artificial polypeptide synthesis, etc. The fragment can be an internal fragment or a C-ter or N-ter fragment of wild-type EHV4-TK, or a combination thereof. Preferably, a fragment according to the present invention retains at least 40%, more preferably at least 50% of the entire amino acid sequence of wild-type EHV4-TK. In a preferred embodiment, a fragment comprises at least 75% of the entire amino acid sequence of wild-type EHV4-TK, more preferably at least 85% thereof. Variants of wild-type EHV4-TK comprise any biologically active polypeptide derived from wild-type EHV4-TK by any mutation(s), substitution(s), addition(s) and/or other modification(s) of the primary or secondary structure. Variants include any natural variants resulting from mutations or polymorphism in EHV4, as well as, for instance, hybrid polypeptides comprising EHV4-TK fused to a heterologous amino acid sequence. The term heterologous means any amino acid sequence comprising one or several amino acid residues, having an origin different from EHV4. Such a heterologous amino acid sequence can be any artificially created sequence providing for instance for an increased stability, or a secretion of the polypeptide, or a marking of the polypeptide. In this regard, the present invention also discloses fusion polypeptides comprising an EHV4-TK moiety fused to a marker protein such as the Enhanced Green Fluorescent Protein (E-GFP), Luciferase, β-Gal, Thy-1 protein, chloramphenicol-acetyl-transferase (CAT), and cell proteins conferring resistance to antibiotic(s). The fusion is generally prepared by genetically coupling a nucleic acid region coding for the EHV4-TK polypeptide and a nucleic acid region coding for the heterologous amino acid sequence, e.g., the marker moiety. The nucleic acids are fused in frame, so that the two regions are expressed simultaneously and yield a chimeric polypeptide.

Mutation or substitution variants may be prepared by known techniques such as specifics mutagenesis, amplification with specific primers and the like. Deletions may also be introduced by conventional techniques using for instance restriction enzymes. Preferably, the variants retain at least 80% of the primary structure of wtEHV4-TK. More preferred variants contain at most 5 modified amino acid residues compared to the wtEHV4-TK.

Other variants may comprise unnatural glycosylation or other post-translational modifications.

As indicated, the variants of EHV4-TK may comprise only one or several modifications as described above (deletion(s), mutation(s), addition(s), etc.).

The biological activity of the above fragments or variants of wild-type EHV4-TK can be assessed by conventional techniques. In particular, the ability of said polypeptides to phosphorylate nucleoside analogs can be measured by contacting said polypeptide with a nucleoside analog such as Acyclovir or Gancyclovir and detecting the presence of phosphated Acyclovir or Gancyclovir. Preferably, the test is conducted by (i) introducing, in a cell, a nucleic acid construct encoding the polypeptide under conditions allowing expression of said polypeptide in said cell, (ii) contacting said cell with a nucleoside analog such as Acyclovir or Gancyclovir, and (iii) assessing the toxicity of the nucleoside analog on said cell. If appropriate, the level of toxicity observed can be compared to a standard toxicity level observed in a similar experiment with a nucleic acid encoding wild-type EHV4-TK. As an example, said test can be performed with any dividing cell such as fibroblasts, for instance. Preferred biologically active fragments or variants retain 75% at least of the toxicity of wild-type EHV4-TK in a test as disclosed above.

As indicated above, the present invention can be performed by using an Equine herpes virus type-4 (EHV4) thymidine kinase polypeptide or a nucleic acid coding therefor. The use of a nucleic acid represents a preferred embodiment. Nucleic acids of this invention can be DNA or RNA, preferably cDNA or an artificial DNA comprising synthetic regions. The nucleic acid encoding wild-type EHV4-TK has been cloned and sequenced.¹⁸ A nucleic acid encoding a EHV4-TK polypeptide of this invention can be obtained by conventional techniques such as library screening, artificial synthesis, cloning from genomic DNA, etc. A particular nucleic acid encoding a EHV4-TK polypeptide of this invention is any nucleic acid which hybridizes with the nucleic acid encoding wild-type EHV4-TK and codes for a biologically active EHV4-TK polypeptide as defined above. Hybridization is preferably performed under stringent conditions, which are known to the skilled artisan (Sambrook et al.), such as for instance 40°C, 50% formamide, 5 x SSC; 1 x Denhardt, with washings at between 55 and 65°C. Another particular nucleic acid of this invention is a nucleic acid encoding wild-type EHV4-TK modified so as to improve the expression conditions. In particular, a preferred nucleic acid of this invention is modified to inactivate any cryptic promoter contained therein. Indeed, it has been observed that thymidine kinase genes contain cryptic promoters which interfere with their expression in mammalian cells. In particular, the presence of cryptic promoters often leads to the expression of truncated versions of thymidine kinase, with lower or no biological activity, and potential side effects (in this respect, see references 19-20). A preferred nucleic acid of this invention is a nucleic acid encoding a EHV4-TK polypeptide, which nucleic acid is devoid of a cryptic promoter. Such a nucleic acid can be prepared by (i) identifying the cryptic promoter in the nucleic acid sequence and (ii) modifying the nucleic acid sequence, for instance by translationally neutral modifications and/or by deletion(s). The resulting nucleic acid can be tested for improved expression in mammalian cells, in particular in human cells, as disclosed in the examples. A particular nucleic acid of this invention comprises a deletion in the 5' region of a EHV4-TK gene, said deletion providing for an increased expression in mammalian cells.

Preferably, the nucleic acid encoding a EHV4-TK polypeptide of this invention is included in a nucleic acid construct comprising for instance a promoter region, located 5' of the coding sequence, and a termination signal located 3' thereof. Such a nucleic acid construct can be part of a vector, such as a plasmid vector, a viral vector (i.e., a recombinant virus), an artificial chromosome, an episome, etc.

In a preferred embodiment, the invention uses a vector comprising a nucleic acid encoding a EHV4-TK polypeptide as defined above. Even more preferably, such a vector is a plasmid or a viral vector, such as a retroviral vector, an adenoviral vector, an AAV, an herpesvirus vector or a vaccinia virus vector. In this regard, one particular embodiment of the invention uses a culture supernatant comprising a recombinant virus which contains a nucleic acid encoding a EHV4-TK polypeptide. Another particular way of providing a nucleic acid of this invention comprises the use of cells which produce a recombinant virus containing a nucleic acid encoding a EHV4-TK polypeptide. In this regard, any composition comprising virus packaging cells can be used, in particular retrovirus or adenovirus packaging cells. The use of a viral supernatant or a virus packaging cell represents a preferred way of carrying out the present invention.

As indicated above, the invention resides in rendering human cells sensitive to a nucleoside analog. selected from ganciclovir and acyclovir The term nucleoside analog means any compound which, when phosphorylated, can be incorporated into an elongating DNA chain to block DNA replication and/or transcription. A nucleoside analog of this invention is a compound that can be converted into a monophosphated nucleoside analog by a thymidine kinase, in particular by HSV-1 TK. Examples of nucleoside analogs include for instance gancyclovir (GCV), acyclovir (ACV), bromovinyl-deoxyuridine (BVDU). These nucleoside analogs are efficiently converted to their monophosphated form by HSV-1 TK, and are then converted to triphosphorylated compounds by host cellular kinases. Incorporation of these compounds into elongating DNA blocks elongation leading to an apoptotic cell death (Balzarini, 1989 ; Furman, 1980 ; St Clair, 1987). Other examples of nucleoside analogs are all compounds derived from pyrimidine or purine nucleosides that can be phosphorylated by EHV4-TK (known antiviral drugs or synthetic products).

The invention thus provides alternative means of rendering cells sensitive to nucleoside analogs selected from ganciclovir and acyclovir. Sensitivity means that, in the presence of said nucleoside analogs, the treated cells will be destroyed or killed, generally by apoptosis. The invention therefore also provides an improved method of destruction of targeted cells. One advantage of the present invention resides in the unexpectedly high sensitivity of the treatment that can be obtained. Indeed, the inventors have now shown that when polypeptides or nucleic acid constructs of the instant invention are contacted with human cells, a very efficient destruction of said cells can be obtained in the presence of a nucleoside analog. In particular, the results presented in the examples show that human cells expressing a EHV4-TK polypeptide are from 3 to 12 fold more sensitive to nucleoside analogs such as GCV than human cells expressing HSV1-TK. In other words, the invention allows to significantly reduce the doses of nucleoside analogs, e.g., GCV, which are used to destroy the target cells.

In this regard, another object of the present invention resides in the use of an EHV4-TK polypeptide or a nucleic acid coding therefor, for the manufacture of a composition for the destruction (e.g., killing) of human cells (in vitro, ex vivo or in vivo). The invention also lies in a method of killing human cells comprising contacting said human cells in vitro, or ex vivo with a EHV4-TK polypeptide or a nucleic acid coding therefor, and further contacting said cells or their progeny with a nucleoside analog.

The present invention can be used in vitro, in vivo or ex vivo. It can be used to kill determined cells, either for selection purposes, depletion purposes, experimental uses or therapeutic applications.

The present invention can be performed with various types of human cells, including naturally, pathologically, or induced diving cells. Naturally dividing cells are cells which divide regularly during their life cycle. Induced dividing cells are cells which would not divide naturally but can be stimulated to divide. A preferred therapeutic use of the instant invention lies in the treatment of proliferating human cells, more preferably abnormally proliferating human cells. A particular illustration is the treatment (i.e., the destruction and/or killing) of human tumor cells. In this regard, various solid tumor cells can be mentioned, such as, for instance, head and neck cancer, lung cancer (in particular non small cell lung cancer), glioblastoma, melanoma, breast cancer, liver cancer, ovarian cancer, prostate cancer, brain tumors and the like. Another example of human cells which can be treated according to this invention is represented by specific clones of T lymphocytes which proliferate abnormally, for instance clones of T lymphocytes involved in autoimmune diseases or in graft versus host disease.

Accordingly, in a particular embodiment, the invention resides in the use of an Equine herpes virus type-4 (EHV4) thymidine kinase polypeptide or a nucleic acid coding therefor for the manufacture of a composition for the killing of human tumor cells in vitro, ex vivo or in vivo. The present invention also lies in a method of killing a human tumor cell comprising contacting said human tumor cell in vitro, ex vivo with an Equine herpes virus type-4 (EHV4) thymidine kinase polypeptide or a nucleic acid coding therefor, and further contacting said cells or their progeny with a nucleoside analog.

In another particular embodiment, the invention resides in the use of an Equine herpes virus type-4 (EHV4) thymidine kinase polypeptide or a nucleic acid coding therefor for the manufacture of a composition for the killing of human T lymphocytes in vitro, ex vivo or in vivo. A method of killing a human T lymphocyte comprising contacting said human T lymphocyte in vitro, ex vivo with an Equine herpes virus type-4 (EHV4) thymidine kinase polypeptide or a nucleic acid coding therefor, and further contacting said cells, or their progeny, with a nucleoside analog is also part of the invention.

As explained above, the present invention can be used in vitro, in vivo or ex vivo. For in vitro or ex vivo uses, the invention can be carried out according to the following procedure.

A population of cells to be treated (i.e., rendered sensitive to a nucleoside analog) is provided. The cells can be maintained in culture in any appropriate device (e.g., a plate, dish, flasc, bottle, etc.), preferably under sterile conditions, in any conventional culture medium (RPMI, DMEM, etc.). The cells are then contacted with the EHV4-TK polypeptide or nucleic acid as defined above. In this regard, the cells can be contacted by direct incubation of the cells with the EHV4-TK polypeptide or nucleic acid. This incubation allows the EHV4-TK polypeptide or nucleic acid to be taken up by the cells, or at least a portion of the cells. This incubation can be performed in the presence of facilitating agents or conditions, which increase the uptake of the EHV4-TK polypeptide or nucleic acid by the cells. Such agents are for instance any cationic lipid, polymer, liposome, calcium phosphate precipitation, etc. The incubation can also comprise physical treatments such as electrical fields, gene gun, and the like. In a particular embodiment of this invention, a EHV4-TK nucleic acid is used, contained in a vector. In a more preferred embodiment, the nucleic acid is contained in a viral vector (or a recombinant virus produced therefrom). The use of a recombinant virus is particularly advantageous because of the natural infectious power of these particles. Preferred recombinant viruses include recombinant retroviruses, adenoviruses, AAV or herpesviruses, for instance. Methods of preparation of these vectors are known to the skilled artisan.

In a particular embodiment, the cells are thus rendered sensitive to a nucleoside analog by contacting said cells with a vector comprising an EHV4-TK nucleic acid, more preferably a recombinant virus comprising an EHV4-TK nucleic acid.

In this respect, the invention relates more particularly to a method of rendering a human cell sensitive to a nucleoside analog selected from ganciclovir and acyclovir, in vitro, ex vivo or in vivo, by contacting the human cell with a vector comprising an EHV4-TK nucleic acid, more preferably a recombinant virus comprising an EHV4-TK nucleic acid. The cells which, upon the incubation, have incorporated the EHV4-TK nucleic acid are sensitive to a nucleoside analog, as well as other surrounding cells, due to the by-stander effect of TK.

Contacting the cells with a recombinant virus as disclosed above can be accomplished by incubation of the cells with a culture supernatant, or an enriched solution or suspension, such as a purified solution, comprising the recombinant virus, or by coculture of the cells with producer cells, which produce the recombinant virus.

The contacting step can last for periods of time of up to 72 hours or more, depending on the incubation conditions. The cells can then be recovered according to any known methods. If desired, they can be subjected to a selection step in order to obtain a population of cells comprising essentially cells which actually contain the EHV4-TK polypeptide or nucleic acid. In this regard, depending on the incubation conditions and the cell population, the percentage of transfected cells can vary from 5 to almost 99%, before any selection step. The recovered cells can be washed and placed in fresh medium, or stored in any appropriate conditions (freezing, lyophilisation, etc.).

For killing the treated cells, the cells are then further contacted with a nucleoside analog selected from ganciclovir and acyclovir i.e., a compound that is converted by the treated cells into a toxic molecule (by incorporation into elongating DNA). For in vitro use, the cells can be contacted at any appropriate dosage of the nucleoside analog, in any suitable device. The treatment can be performed to screen for proliferating cells, eliminate specific cell populations (i.e., depletion), assess the activity of a promoter region, etc. For ex vivo indications, the nucleoside analog treatment can also be performed once the treated cells have been injected in a subject. In this embodiment, the dosages are essentially as discussed below for in vivo uses.

Indeed, for performing the invention in vivo, cells are to be contacted with the EHV4-TK polypeptide or nucleic acid by direct administration to a subject in need thereof. Administration can be performed by any appropriate route (intra arterial, intravenous, intra muscular, intra tumoral, cutaneous, etc.). For treatment of proliferating cells, in particular tumor cells, the EHV4-TK polypeptide or preferably, nucleic acid, is preferably administered by intravenous, intra arterial, or intra tumoral injections, more preferably intra tumoral injection, according to known techniques. The dosage of EHV4-TK nucleic acid can be adjusted by the skilled artisan, based on the teaching of the prior art clinical trials with HSV1-TK gene.

In this respect, nucleic acids can be injected from 0,1 to 1000 µg/dose, for instance. With regard to recombinant virus, 10³ - 10⁹ pfu can be injected at dose. When packaging cells are being used, 10⁴ - 10⁹ cells are preferably injected.

Once the target cells have become sensitive to a nucleoside analog, they can be killed by further contact with a nucleoside analog.

In this regard, the nucleoside analog can be administered to the subject according to various protocols and different timeframes. Generally, the nucleoside analog is administered by perfusion, intra arterial, intravenous, or cutaneous routes. Conventional dosage for nucleoside analogs range from 1 to 50 mg/kg per day, in particular about 10 mg/kg, twice a day. The nucleoside analog can be GCV or ACV. Oral administration is also envisioned. For treatment of tumor cells for instance, the nucleoside analog is usually administered shortly after the EHV4-TK polypeptide or nucleic acid, and for a period of time comprised between 1 to 15 days. For other cells, such as specific T cell clones, the nucleoside analog is to be administered only when these particular targeted T cell clones proliferate, for instance (i.e., GVHD, auto immune diseases, etc.)

The invention also relates to a pharmaceutical composition for the treatment of human cell proliferative disorders comprising an Equine herpes virus type-4 (EHV4) thymidine kinase polypeptide or a nucleic acid coding therefor and a pharmaceutically acceptable excipient. In a preferred embodiment, the pharmaceutical composition comprises a vector comprising the above nucleic acid, such as a viral vector as defined above. In another embodiment, the pharmaceutical composition comprises a recombinant virus producing cell, such as a retrovirus producing cell, for instance.

Preferably, the pharmaceutical composition of the instant invention further comprises a nucleoside analog selected from ganciclovir and acyclovir, to be administered simultaneously or separately.

Also described is a composition comprising human cells containing an EHV4-TK nucleic acid as defined above.

Other aspects and advantages of the present invention will be presented in the following experimental section, which should be regarded as illustrative and not limiting the scope of the invention.

### LEGEND TO THE FIGURES

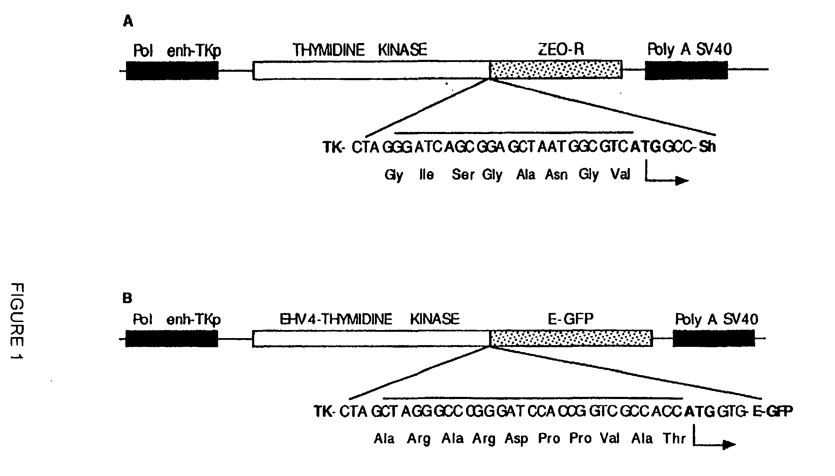

### Figure 1: Map of the expression vectors.

(A) Plasmids as described in Cazaux et al. harbor the cDNA encoding an herpesvirus TK (HSV1, EHV4, EBV or VZV) fused in frame with the *She ble* gene conferring resistance to zeocin. (B) Map of the vector coding for the fusion protein EHV4-TK/E-GFP. Underlined sequences correspond to the 5' to 3' arm between the thymidine kinase and the Zeocin resistance protein or the E-GFP.

### Figure 2: Analysis of fusion TK/ZEO protein expression in transfected cells.

(A) Western blot analysis showing HSV1- and EHV4-TK protein levels in tk(-) 3T3 stable clones. (B) Dot blot analysis of TK expression in transfected and non-transfected tk(-) 143B cells. Fusion proteins were revealed with purified rabbit anti-Sh polyclonal antibody.

### Figure 3: Cytotoxic activity of ACV, GCV and BVDU against human tk(-) 143B cells transfected with HSV1- or EHV4-TK. Toxicity is presented as mean of IC50 values ± SD. The means, determined on 3 independent experiments, are indicated on the top of each histogram.

### Figure 4: Thymidine and GCV phosphorylation in transfected and non-transfected tk(-) 143B cells.

Cells were incubated with radiolabeled thymidine or GCV, and the different phosphorylated forms were separated by FPLC analysis. The percentage of thy-MP, thy-DP and thy-TP were respectively 29.3, 27.5 and 39.2% in HSV1-TK expressing cells and 36.8, 27.5 and 28.8% in EHV4-TK expressing cells. The percentage of GCV-MP, GCV-DP and GCV-TP were respectively 15.5, 36.5 and 45% in HSV1-TK expressing cells and 23.9, 29.3 and 45% in EHV4-TK expressing cells.

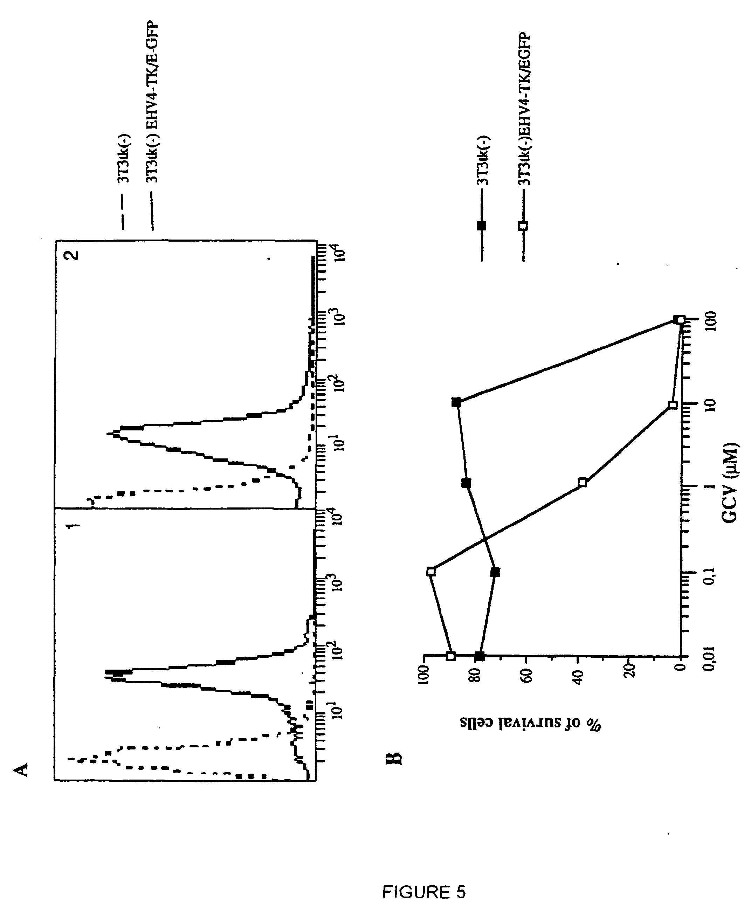

### Figure 5: Analysis of the dual functionality of the EHV4-TK/E-GFP fusion protein.

(A) E-GFP expression in tk(-) 3T3 transfected and non-transfected cells. EHV4-TK gene fused with E-GFP gene (Figure 1-B) was transfected in tk(-) 3T3 cells, and E-GFP positive cells were detected by FACS analysis. Expression of E-GFP was determined a week after transfection (1) and after 2 months of culture in HAT medium (2). (B) GCV toxicity in transfected and non-transfected cells. Results were expressed as % of viable cells compared to cells grown without GCV.

### MATERIALS AND METHODS

### Chemicals

Acyclovir (ACV, [9-(2-hydroxyethoxymethyl)guanine] Zovirax®), Ganciclovir (GCV, [9-(1,3-dihydroxy-2-propoxymethyl)guanine] Cymevan®), Bromovinyldeoxyuridine, (BVDU (E)-5-(2-bromovinyl)-2'-deoxyuridine), were purchase from Wellcome ( Issy les-Moulineaux, France), Syntex (Palo Alto, CA, USA) and Sigma Chemical Co. (St Louis, MO, USA), respectively.

### Cell lines and Plasmids

Murine NIH 3T3 cells lacking an endogenous TK (tk(-) 3T3) activity were obtained from the American Type Culture Collection (Rockville, MD, USA) as well as human tk(-) 143B (ATCC CRL 8303) and HCT116 (ATCC CCL 247) cells; murine pancreatic tumor cell line, PANC-O2, was a gift from Rhone-Poulenc (France). All cells were grown in Dubelcco's modified Eagle's medium (DMEM) supplemented with 1% L-glutamine (GibcoBRL), 1% penicillin, streptomycin and neomycin mixture (GibcoBRL) and 10% new born calf serum (GibcoBRL) for NIH 3T3 cells or 10% fetal calf serum (GibcoBRL) for other cell lines.

Expression vectors harboring the cDNA encoding herpesvirus TK (HSV1, EHV4, EBV or VZV) fused in frame with the *Sh Ble* gene conferring resistance to zeocin, have been previously described by Cazaux et al.²¹ The fused genes are under the transcriptional control of the HSV1-TK viral promoter and the viral polyoma pYF441 enhancer (Figure 1-A).

Plasmid containing HSV1-TK gene fused in frame with the E-GFP gene (Figure 1-B) was constructed from plasmid described by PCR replacement of the *Sh Ble* gene with the E-GFP sequence (Clontech, Palo Alto, California, USA).

### DNA Transfection:

Cells were transfected by the various plasmids using a calcium phosphate precipitation kit (GibcoBRL). After 48 h of phenotypic expression, cells were grown in selective medium with zeocin (Cayla, Toulouse, France): 25 µg/ml (3T3 cells), 50 µg/ml (143 B cells) or 100 µg/ml (PANC-O2 and HCT 116 cells).

Transfection was performed in 2 petri-dishes (Ø 100mm). Independent clones were picked-up and individually expanded, or were left in culture to obtain a cell bulk.

### Cytotoxicity assay:

ACV, GCV and BVDU toxicity were quantitatively assessed by measuring inhibition of cellular proliferation. Cells were seeded at 2.5x10³ per wells in 24-wells plates. 24 h later they were grown in drug-containing medium for 7 days. Drug concentrations ranging from 0.001 nM to 100 µM were tested in triplicate. The medium was changed with fresh DMEM every 2 days. On day 6, 1.5 µCi of [methyl-³H]thymidine (Amersham, UK) was added per well. After washes with phosphate-buffered saline (PBS pH 7.3), incorporated radioactivity was determined with a Micro-Beta Plus counter (EG&G Instruments and Wallac, Turku, Finland). Cytotoxic doses were expressed as IC50 values (the drug concentration required to reduce the [methyl-³H]thymidine incorporation by 50%), and selectivity indexes were calculated as the IC50 ratios of parental to transfected cells.

### Viability assay:

Parental and viral-TK expressing cells were plated at 5x10³ per wells in 6-wells dishes. After 24 h, drugs was added to the medium at various concentrations ranging from 0.01 to 100 µM. Medium containing drugs was changed every 2 days. After 7 days of culture, cell viability was assayed by counting viable cells by trypan blue exclusion after 7 days of culture with GCV.

### Western Blot analysis:

Cells were washed three times in ice-cold PBS, and 10⁶ transfected cells were incubated in lysis buffer ³⁷ Lysates from 1x10⁵ cells were loaded on each line on a 10-12% trycine-SDS polyacrylamide gel electrophoresis (Novex, San Diego, CA, USA), and separated proteins were transferred onto nitro-cellulose membrane (Schleicher & Schuell, Dassel, Germany) by electroblotting. To ensure equivalent protein loading in each line and correct transfer, bands were made visible by staining with 0.3% Ponceau Rouge staining. Zeo-fusion proteins were revealed with purified rabbit anti-Zeo polyclonal antibodies (Cayla, Toulouse, France) followed by incubation with antibody to rabbit IgG, conjugated to horseradish peroxydase. The reaction was visualized using an BM chemiluminescence detection method (Boehringer Mannheim, France SA).

### Dot Blot analysis:

Successive dilutions of cellular extracts were dropped on nitro-cellulose membrane. Zeo-fusion proteins were revealed with rabbit anti-Zeo polyclonal antibodies (Cayla, Toulouse, France) followed by incubation with antibody to rabbit IgG conjugated to horseradish peroxydase (Boehringer Mannheim, France SA). The reaction was visualized as described in Western Blot analysis.

### Nucleotide analysis by fast protein liquid chromatography(FPLC):

3x10⁶ cells were incubated for 7 hours with 1 µM of [8-³H] GCV (3 Ci/mmol, Isotopchim, France) or [methyl-³H] Thymidine (5 Ci/mmol, Amersham, UK) in a final volume of 5 ml of medium. Cells were detached by trypsin treatment, and washed twice with PBS. The nucleotides were extracted with 1 ml of ice cold methanol 60% over the night at -20°C. The samples were analyzed by fast liquid chromatography (FPLC, Pharmacia-LKB Instruments, Sweden) on a anion-exchange column.^{38,39} Radioactivity was counted at the column exit with a radiamatic Flo-one Beta A-500 apparatus (Packard, Meriden, CT), and peaks were analyzed with Flo-one/Data software.

### Flow cytometric analysis:

tk(-) 3T3 cells were transfected with plasmid encoding the EHV4-TK/E-GFP fusion protein, and selected in HAT medium. After 1 week of culture, fluorescence of transfected cells were analyzed. The fluorescence analysis was repeated 2 months after selection. Cells were washed twice with PBS, and intensity of fluorescence was directly determined on a FACSCalibur (Becton Dickinson, Sunnyvale, CA) with a 525 nm filter and analyzed with CellQuest software.

### RESULTS

### Nucleoside analogs toxicity on cell bulks expressing different herpesviruses thymidine kinases

We used expression vectors with herpesvirus TK genes fused in frame with the *Sh Ble* gene conferring resistance to zeocin (pUT-TKx) (Figure 1A).²¹ The use of such vectors facilitates the selection of transduced cells and the detection of the chimeric protein by immunodetection with anti-*Sh* polyclonal antibodies. The functionality of the viral TK expressed by these vectors has been previously demonstrated in bacteria and yeast lacking endogenous TK or thymidylate kinase activities.²¹ We first expressed these genes in murine fibroblasts deficient in cellular TK (tk(-) 3T3 cells) and selected the stable transfected cells based on their resistance to zeocin. The functionality of the viral TK in cells was then tested by analyzing cell growth in selective HAT medium and by [methyl-^{3H}]thymidine (³HT) incorporation assays. As expected, while all untransfected cells died in HAT, all transfectants survived and incorporated ³HT (data not shown).

Selected bulk populations were then analyzed for their *in vitro* sensitivity to nucleoside analogs by measuring inhibition of cell proliferation in comparison with NIH-3T3 parental cells (Table 1). ACV IC50 values were approximately fifteen fold lower in HSV1-, EHV4- and VZV-TK expressing cells compared to the parental NIH-3T3 cells, and only four fold lower for EBV-TK expressing cells. HSV1- and EHV4-TK expressing cells were 350 and 700 fold more sensitive to GCV than NIH-3T3 cells, compared to only 7 fold for VZV- or EBV-TK expressing cells. Unlike ACV and GCV, IC50 values for BVDU were in the same range for all herpesvirus-TK expressing cell bulks, about 1300 to 3600 fold lower than for NIH-3T3 cells.

These observations were extended using an additional murine tumor cell line, the pancreatic tumor PANC-O2 cells. Again, it was found that_IC50 values were 3 fold lower for EHV4-TK than for HSV1-TK expressing cell bulk, 0.55 ± 0.07 and 1.7 ± 0.5 µM respectively (means of two independent experiments, data not shown).

**Table 1****: Cytotoxicity of ACV, GCV and BVDU on bulk transfected and non transfected murine tk(-) 3T3 cells.** Drug toxicity was assessed by measuring inhibition of cell proliferation. Cells were grown in drug-containing medium for 7 days. [methyl-³H]-thymidine was added at day 6 and incorporated radioactivity was measured. Cytotoxic doses were expressed as mean IC50 values (50% inhibitory concentration required to reduce the [methyl-³H]-thymidine incorporation by 50%) of 2 or 3 experiments and selectivity indexes were calculated as the IC50 ratios of parental to transfected cells.

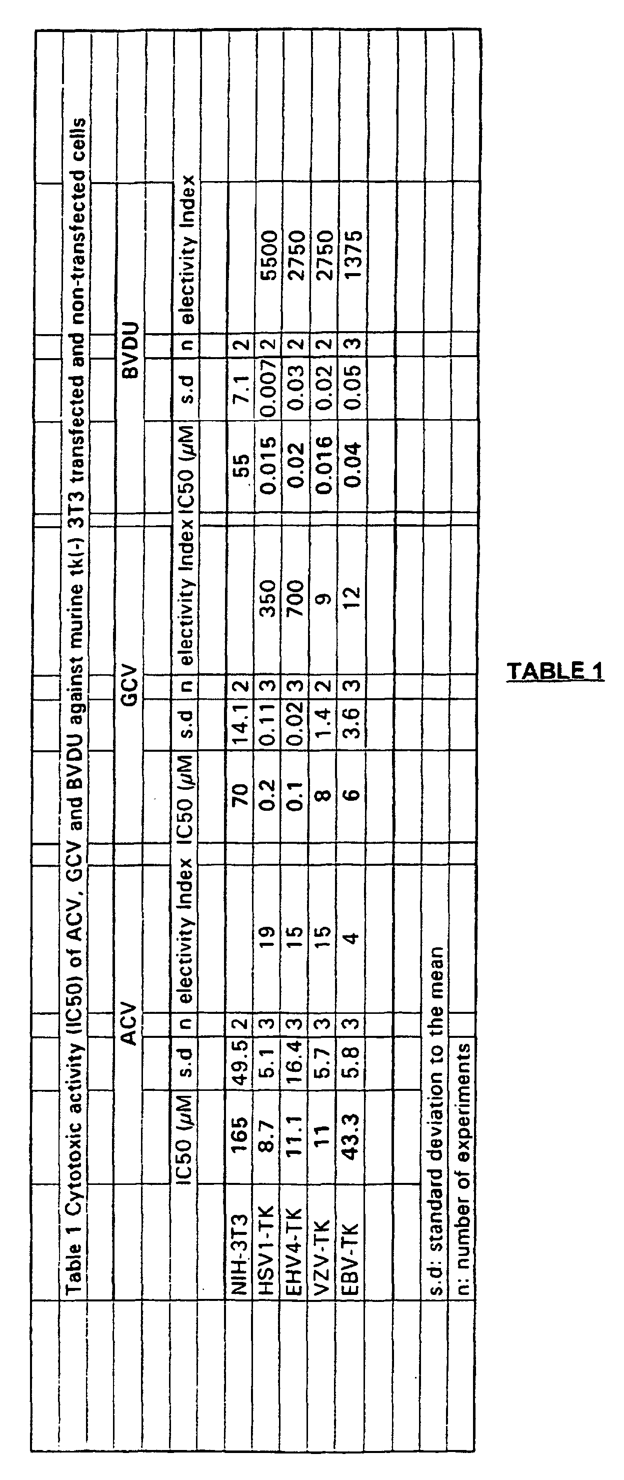

### Nucleoside analog toxicity on HSV1-TK or EHV4-TK expressing cell clones.

Among the four herpesviruses TK tested for their capacity to sensitize cells to nucleoside analogs toxicity, HSV1- and EHV4-TK demonstrated the more interesting selectivity indexes. Because cell bulks represent a mixture of clones with different sensibilities, we next analyzed the drug cytotoxicity on individual clones expressing either of these two TK.

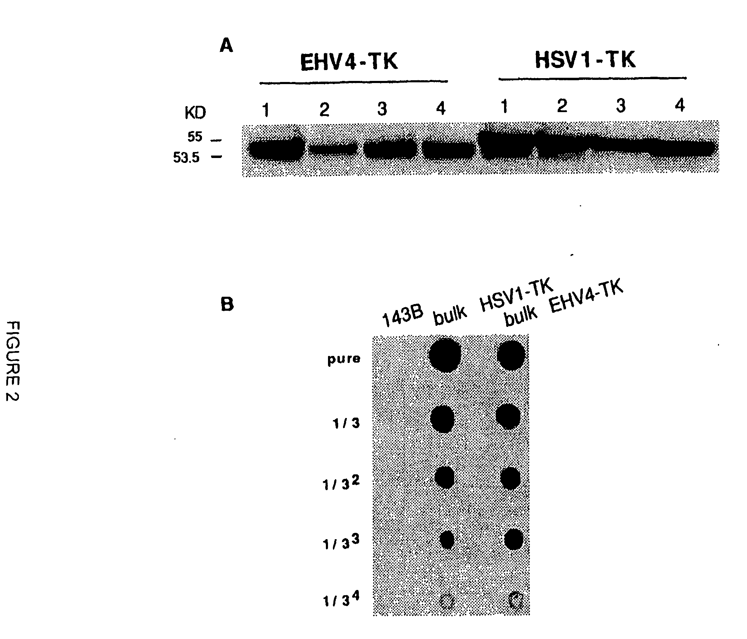

We first analyzed the herpesvirus-TK protein expression level for several clones by western-blot using polyclonal antibodies against *Sh* protein. Cellular extract of 10⁵ cells were loaded to electophoresis. Bands corresponding to EHV4-TK/ZEO (53 KDa), and to HSV1-TK/ZEO (55 KDa) were observed with similar intensity in the four EHV4-TK+ and four HSV1-TK+ clones tested (Figure 2-A). There was no detectable band corresponding to Sh protein alone (20Kda) in these cell extracts (data not shown). A weaker band of lower PM could also be observed with all extracts of HSV1-TK(+) cells. This band corresponds to a transcription initiation at a known cryptic internal promoter resulting in the translation of a truncated but functional protein.^{19-20,22}

We then analyzed the toxicity of ACV, GCV and BVDU, on these eight clones (Table 2). For ACV and GCV, the sensitivity of EHV4-TK expressing cells was statistically more important than the one of HSV1-TK expressing cells (p < 0.05 and 0.01 respectively). HSV1- or EHV4-TK expressing clones displayed the same BVDU sensitivity.

**Table 2****: Cytotoxicity of ACV, GCV and BVDU on HSV1-TK and EHV4-TK expressing murine tk(-) 3T3 clones.** Drug sensitivity of TK expressing clones were tested as described in Table 1. Cytotoxic doses were expressed as mean IC50 values of 3 experiments.

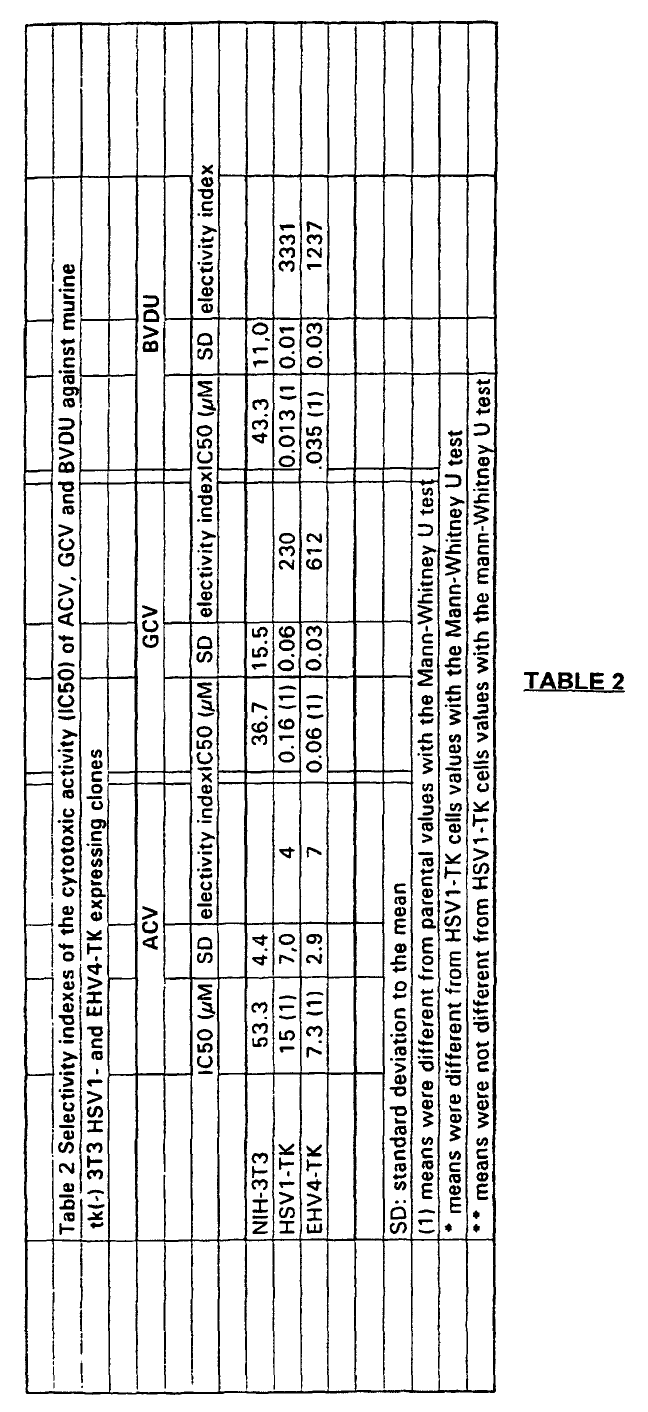

### Nucleoside analog toxicity on human cell bulk expressing HSV1-TK or EHV4-TK.

Since nucleoside analog's metabolism might be different in cells of different species ²³⁻²⁵ we thus analyzed the efficiency of the system for killing human cells.

Human tk(-) osteosarcoma 143B cell bulks expressing HSV1- or EHV4-TK were thus generated. We first analyzed the herpesvirus-TK protein expression levels for each cell bulks by dot-blot using polyclonal antibodies against *Sh* protein (Figure 2-B). There was no detectable band corresponding to fused protein in tk(-) 143B cell extracts. Similar expression of the fused protein was observed in HSV1 or EHV4-TK expressing cells.

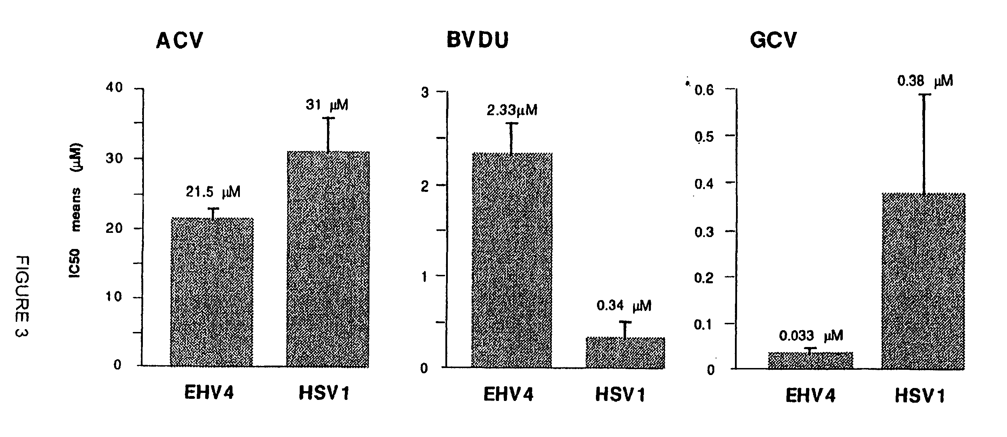

We then, studied cell sensitivity to ACV, GCV and BVDU (Figure 3). The toxicity of ACV was similar in HSV1- and EHV4-TK expressing cells. Noteworthy, EHV4-TK expressing cells were approximately twelve fold more sensitive to GCV than HSV1-TK expressing cells. On the contrary the toxicity of BVDU was greater in HSV1-TK than in EHV4-TK expressing cells (Figure 3).

These observations were extended to an additional tumor cell line, human colonic carcinoma HCT116 cells. IC50 values for GCV were 3 fold lower for EHV4-TK than for HSV1-TK expressing cells, 0.11 ± 0.02 and 0.32 ± 0.08 µM respectively (means ± SD for 3 independent experiments). EHV4- and HSV1-TK expressing cells were 485 and 170 fold more sensitive to GCV than HCT116 cells (mean of IC50 values for GCV was 53.4 ± 15 µM).

### GCV phosphorylation in human cells expressing HSV1- or EHV4-TK.

In order to understand why GCV is more toxic in EHV4-TK compared with HSV1-TK expressing cells, we analyzed the GCV phosphorylation in tk(-) 143B and HSV1- or EHV4-TK expressing cells. After 7 hours of incubation with radiolabeled GCV or thymidine, nucleotides were extracted and analyzed by FPLC.

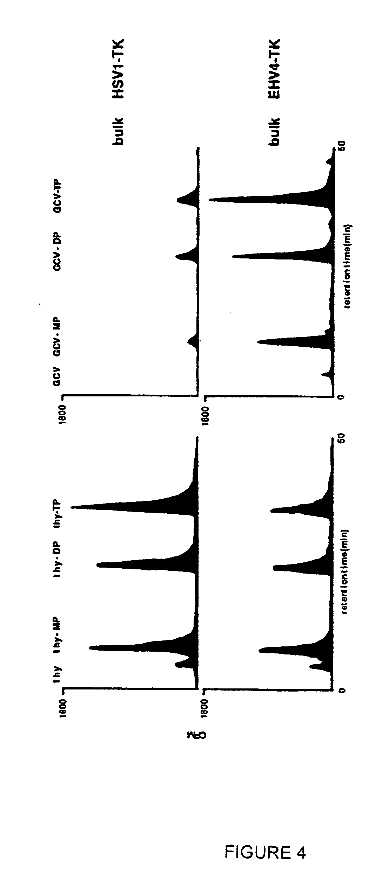

While there was no detectable phosphorylation of thymidine in tk(-) 143B cells (data not shown), thymidine was similarly phosphorylated in both TK expressing cells (Figure 4). For GCV, the proportions of the different phosphorylated forms were the same in both TK cells, while the total amount of intracellular GCV was 5 fold higher for EHV4 than for HSV1-TK expressing cells.

### Functionality of an EHV4-TK/E-GFP fusion protein.

To facilitate the transgene detection in transfected cells or tissues, the E-GFP gene was fused in frame at the 3' end of the EHV4-TK gene and cloned into an expression vector (Figure 1-B). This construction was then transfected into tk(-) 3T3 cells, followed by FACS analysis of E-GFP positive cells. A cell bulk containing 95% of E-GFP expressing cells was obtained. The stable expression and the dual functionality of the transgene could be demonstrated by analyzing fluorescence and survival in presence of HAT medium during 2 months of culture (Figure 5-A). Expression of the EHV4-TK/E-GFP fusion protein efficiently sensitized these cells to GCV for which the IC50 was 0.6 µM, compared to 30 µM for tk(-) 3T3 cells (Figure 5-B).

### DISCUSSION

We aimed to improve the efficacy of current herpes virus derived TK systems for cell destruction. For that purpose, we chose to assess the efficacy of alternative TK genes in human cells. Since we needed to monitor the expression level of these genes, they were fused to *sh ble*, encoding a protein conferring resistance to zeocin, which can be detected by western blotting with available polyclonal antibodies.

We first verified the dual functionality of the different TK-ZEO fusions in eukaryotic cells by transfection of murine or human cells deficient for endogenous thymidine kinase activity. Selection with zeocin always generated resistant clones that all survived when later cultured in the presence of HAT media. Therefore we concluded that even if peptidic fusions were not cleaved into the cells, all the fusion proteins were bi-functional. This is in agreement with previous experiments showing that HSV1-TK retain its functionality after amino- or carboxy-terminal fusion to various proteins.^{21, 24, 26-28}

Significant differences in the activity of the different herpes TK were observed. These differences were found both when analyzing cell bulks as well as independent clones. The bulk provides a better estimate of the overall enzyme activity in a polyclonal population, that mimics the *in situ* transfected population during pre-clinical and clinical setting, while the clones allow to analyze the activity in individual cells with respect to transgene expression levels. We studied the toxicity of ACV, GCV and BVDU.

VZV- and EBV-TK did not demonstrate any advantages over HSV1-TK, whatever the nucleoside analog used. Two recent studies showed the potential of VZV-TK as a suicide gene in human breast cancer cells²⁹ and in human osteosarcoma cells.³⁰ In these articles selectivity indexes and IC50 values for BVDU ranged from 0.06 to 0.6 µM, quite similar to our values, but no comparison between VZV and HSV1-TK was shown.

Noteworthy, EHV4-TK expressing cells were always more sensitive to GCV than HSV1-TK expressing ones, whether as bulks or clones. This enhanced toxicity was not due to a difference in TK expression levels as assessed by western blot. This is also further indicated by the observation of an opposite effect with BVDU that is more toxic for HSV1-TK expressing cells, demonstrating a qualitative rather than a quantitative difference between the activity of these enzymes.

To investigate the possible mechanism explaining the improved sensitivity to GCV in EHV4-TK expressing cells compared to HSV1-TK expressing cells, we analyzed GCV phosphorylation by FPLC. The total amount of intracellular GCV was 5 fold higher in EHV4-TK than in HSV1-TK expressing cells, while proportions of the different phosphorylated forms were similar in both TK cells. Such a difference might be explained by the uptake of nucleoside and their analogs that has been reported to be correlated with TK expression. Indeed, the uptake of thymidine into tk(-) *Escherichia coli* is proportional to the amount of TK activity expressed from the heterologous HSV1-TK gene.³¹ Furthermore, mammalian HSV1-TK expressing cells showed higher GCV uptake and phosphorylation than control cells.³² As we detected similar TK expression in both TK(+) cells (Figure 2-B), the greater amount of intracellular GCV in EHV4-TK expressing cells could be correlated to a greater EHV4-TK activity towards GCV compared to HSV1-TK activity.

Gene transfer detection could be greatly facilitated by markers that allow rapid and efficient detection of successfully transduced cells. This specially applies to cytoplasmic proteins. However, no suitable anti-EHV4-TK monoclonal or polyclonal antibodies are available. We thus used the Enhanced-Green-Fluorescent Protein (E-GFP) gene to generate a EHV4-TK/E-GFP fusion protein. Expression of E-GFP remains stable for months in transfected cells and can be easily monitored by flow cytometry. Transfection of the fusion plasmid to mammalian cells revealed that the construct is functional, cells being both fluorescent (95%) and sensitive to GCV. This result is in line with the recent study of Liomas *et al* who reported the dual functionality of a HSV1-TK-green fluorescent protein fusion gene.²⁸ This chimaeric suicide/marker gene should dramatically facilitate the detection of transduced cells. This would be particularly important in experimental models. In this regard, suicide genes are frequently used in transgenic mice to generate models of conditional ablation of specific cell populations.

Depending on the cell lines, EHV4-TK was three to twelve fold more potent than HSV1-TK. Such a difference appears significant when contemplating the clinical use of suicide genes since the treatment of experimental tumors in mice or rats demonstrate that the GCV dosage is often critical for obtaining efficacy. When administered at 10 mg/kg twice a day, the usual dosage in humans, the GCV plasmatic concentration range between 0.44 to 2.2 µg/ml,³³ and the cerebral spinal fluid (CSF) concentrations are approximately 3 fold lower.^{34, 35} It is therefore possible that CSF GCV concentrations are sub-optimal for the treatment of brain tumors transduced with HSV1-TK. In any case, a better efficiency of the enzyme should lead to a better treatment efficiency and/or a decrease of the GCV dose used. This later point is also significant since GCV toxicity in human appears cumulative depending on the overall amounts administered (Cytovene® product monograph).³⁶

Altogether, these experiments underline the potential advantages of the EHV4-TK as a suicide gene, for both experimental, and therapeutical approaches, in vitro, ex vivo or in vivo.

### BIBLIOGRAPHY

1. Anonymous. Department of Health and Human Services National Institutes of Health. Recombinant DNA Advisory Committee. Minutes of Meeting. March 6-7, 1997. *Hum Gene Ther* 1997; **8:** 1955-2004.
2. Fyfe, J. A., Keller, P. M., Furman, P. A., Miller, R. L., and Elion, G. B. Thymidine kinase from herpes simplex virus phosphorylates the new antiviral compound, 9-(2-hydroxyethoxymethyl)guanine. *J Biol Chem* 1978; **253**: 8721-8727.
3. Cheng, Y. C., Dutschman, G., De Clercq, E., Jones, A. S., Rahim, S. G., Verhelst, G., and Walker, R. T. Differential affinities of 5-(2-halogenovinyl)-2'-deoxyuridines for deoxythymidine kinases of various origins. *Mol Pharmacol* 1981; **20**: 230-233.
4. Field, A. K., Davies, M. E., DeWitt, C., Perry, H. C., Liou, R., Germershausen, J., Karkas, J. D., Ashton, W. T., Johnston, D. B., and Tolman, R. L. 9-([2-hydroxy-1-(hydroxymethyl)ethoxy]methyl)guanine: a selective inhibitor of herpes group virus replication. *Proc Natl Acad Sci U S A* 1983; **80**: 4139-4143.
5. Furman, P. A., McGuirt, P. V., Keller, P. M., Fyfe, J. A., and Elion, G. B. Inhibition by acyclovir of cell growth and DNA synthesis of cells biochemically transformed with herpesvirus genetic information. *Virology* 1980; **102**: 420-430.
6. St Clair, M. H., Lambre, C. U., and Furman, P. A. Inhibition by ganciclovir of cell growth and DAN synthesis of cells biochemically transformed with herpesvirus genetic information. *Antimicrob. Agents and Chemother*. 1987; **31:** 844-849.
7. Balzarini, J., and De Clercq, E. Inhibitory effects of (E)-5-(2-bromovinyl)-2'-deoxyuridine (BVDU) and related compounds on herpes simplex virus (HSV)-infected cells and HSV thymidine kinase gene-transformed cells. *Methods Find Exp Clin Pharmacol* 1989; **11**: 379-389.
8. Bouayadi, K., Hoffmann, J. S., Fons, P., Tiraby, M., Reynes, J. P., and Cazaux, C. Overexpression of DNA polymerase beta sensitizes mammalian cells to 2',3'-deoxycytidine and 3'-azido-3'-deoxythymidine. *Cancer Res* 1997; **57**: 110-116.
9. Culver, K. W., Ram, Z., Wallbridge, S., Ishii, H., Oldfield, E. H., and Blaese, R. M. In vivo gene transfer with retroviral vector-producer cells for treatment of experimental brain tumors. *Science* 1992; Using Smart Source Parsing, **256**: 1550-1552.
10. Vile, R. G., and Hart, I. R. Use of tissue-specific expression of the herpes simplex virus thymidine kinase gene to inhibit growth of established murine melanomas following direct intratumoral injection of DNA. *Cancer Res* 1993; Using Smart Source Parsing, **53**: 3860-3864.
11. Caruso, M., Panis, Y., Gagandeep, S., Houssin, D., Salzmann, J. L., and Klatzmann, D. Regression of established macroscopic liver metastases after in situ transduction of a suicide gene. *Proc Natl Acad Sci USA* 1993; **90:** 7024-7028.
12. Wei, M. X., Bougnoux, P., Sacre-Salem, B., Peyrat, M. B., Lhuillery, C., Salzmann, J. L., and Klatzmann, D. Suicide gene therapy of chemically induced mammary tumor in rat: efficacy and distant bystander effect. *Cancer Res* 1998; **58**: 3529-3532.
13. ORDA, R. Human Gene Transfer Protocols. *Office of Recombinant DNA Actvities, NIH, Bethesda, Maryland* 1998; .
14. Ram, Z., Culver, K. W., Oshiro, E. M., Viola, J. J., DeVroom, H. L., Otto, E., Long, Z., Chiang, Y., McGarrity, G. J., Muul, L. M., Katz, D., Blaese, R. M., and Oldfield, E. H. Therapy of malignant brain tumors by intratumoral implantation of retroviral vector-producing cells [see comments]. *Nat Med* 1997; **3:** 1354-1361.
15. Bonini, C., Ferrari, G., Verzeletti, S., Servida, P., Zappone, E., Ruggieri, L., Ponzoni, M., Rossini, S., Mavilio, F., Traversari, C., and Bordignon, C. HSV-TK gene transfer into donor lymphocytes for control of allogeneic graft-versus-leukemia [see comments]. *Science* 1997; **276**: 1719-1724.
16. Klatzmann, D., Chérin, P., Bensimon, G., Boyer, O., Coutelier, A., Charlotte, F., Boccaccio, C., Salzmann, J. L., and Herson, S. A phase-I/II dose-escalation study of herpes simplex virus type 1 thymidine kinase "suicide" gene therapy for metastatic melanoma. *Human Gene Therapy* 1998; 9(**17**): 2585-2594.
17. Klatzmann, D., Valéry, C. A., Bensimon, G., Marro, B., Boyer, O., Mokhtari, B., Diquet, B., Salzmann, J. L., and Philoppon, J. A phase I/II study of herpes simplex type 1 thymidine kinase "suicide" gene therapy for recurrent glioblastoma. *Human Gene Therapy* 1998; 9(**17**):2595-2604.
18. Nicolson L., Cullinane A, and Onions, D. The nucleoside sequence of the equine herpes virus 4 Thymidine Kinase Gene, J. General Virology, 1990, 71, 1801-1805.
19. Al-Shawi, R., Burke, J., Wallace, H., Jones, C., Harrison, S., Buxton, D., Maley, S., Chandley, A., and Bishop, J. O. The herpes simplex virus type 1 thymidine kinase is expressed in the testes of transgenic mice under the control of a cryptic promoter. *Mol Cell Biol* 1991; **11**: 4207-4216.
20. Salomon, B., Maury, S., Loubiere, L., Caruso, M., Onclercq, R., and Klatzmann, D. A truncated herpes simplex virus thymidine kinase phosphorylates thymidine and nucleoside analogs and does not cause sterility in transgenic mice. *Mol Cell Biol* 1995; **15**: 5322-5328.
21. Cazaux, C., Tiraby, M., Loubiere, L., Haren, L., Klatzmann, D., and Tiraby, G. Phosphorylation and cytotoxicity of therapeutic nucleoside analogues: a comparison of alpha and gamma herpesvirus thymidine kinase suicide genes [In Process Citation]. *Cancer Gene Ther* 1998; **5:** 83-91.
22. Ellison, A. R., and Bishop, J. O. Initiation of herpes simplex virus thymidine kinase polypeptides. *Nucleic Acids Res* 1996; **24**: 2073-2079.
23. Balzarini, J., Pauwels, R., Baba, M., Herdewijn, P., De Clercq, E., Broder, S., and Johns, D. G. The *in vitro* and *in vivo* anti-retrovirus activity, and intracellular metabolism of 3'-azido-2',3'-dideoxythymidine and 2',3'-dideoxycytidine are highly dependent on the cell species. *Biochemical Pharmacology* 1988; **37:** 897-903.
24. Beck, C., Cayeux, S., Lupton, S. D., Dorken, B., and Blankenstein, T. The thymidine kinase/ganciclovir-mediated "suicide" effect is variable in different tumor cells. *Hum Gene Ther* 1995; **6:** 1525-1530.
25. Zhang, L., Winkenheiser, K. A., and Whitsett, A. Limitations of retrovirus-mediated HSV-tk gene transfer to pulmonary adenocarcinoma cells in vitro and in vivo. *Human Gene Therapy* 1997; **8**: 563-574.
26. Schwartz, F., Maeda, N., Smithies, O., Hickey, R., Edelmann, W., Skoultchi, A., and Kucherlapati, R. A dominant positive and negative selectable gene for use in mammalian cells. *Proc Natl Acad Sci U S A* 1991; **88**: 10416-10420.
27. Marini, F. r., Cannon, J. P., Belmont, J. W., Shillitoe, E. J., and Lapeyre, J. N. In vivo marking of spontaneous or vaccine-induced fibrosarcomas in the domestic house cat, using an adenoviral vector containing a bifunctional fusion protein, GAL-TEK. *Hum Gene Ther* 1995; **6:** 1215-1223.
28. Loimas, S., Wahlfors, J., and Janne, J. Herpes simplex virus thymidine kinase-green fluorescent protein fusion gene: new tool for gene transfer studies and gene therapy. *Biotechniques* 1998; **24**: 614-618.
29. Grignet-Debrus, C., and Calberg-Bacq, C. M. Potential of Varicella zoster virus thymidine kinase as a suicide gene in breast cancer cells. *Gene Ther* 1997; **4:** 560-569.
30. Degreve, B., Andrei, G., Izquierdo, M., Piette, J., Morin, K., Knaus, E. E., Wiebe, L. I., Basrah, I., Walker, R. T., De Clercq, E., and Balzarini, J. Varicella-zoster virus thymidine kinase gene and antiherpetic pyrimidine nucleoside analogues in a combined gene/chemotherapy treatment for cancer. *Gene Ther* 1997; **4:** 1107-1114.
31. Dube, D. K., Horwitz, M. S., and Loeb, L. A. The association of thymidine kinase activity and thymidine transport in Escherichia coli. *Gene* 1991; **99**: 25-29.
32. Haberkorn, U., Khazaie, K., Morr, I., Altmann, A., Muller, M., and van Kaick, G. Ganciclovir uptake in human mammary carcinoma cells expressing herpes simplex virus thymidine kinase. *Nucl Med Biol* 1998; **25**: 367-373.
33. Oldfield, E. H., Ram, Z., Culver, K. W., Blaese, R. M., DeVroom, H. L., and Anderson, W. F. Gene therapy for the treatment of brain tumors using intra-tumoral transduction with the thymidine kinase gene and intravenous ganciclovir. *Hum Gene Ther* 1993; **4:** 39-69.
34. Fletcher, C., Sawchuk, R., Chinnock, B., de Miranda, P., and Balfour, H. H., Jr. Human pharmacokinetics of the antiviral drug DHPG. *Clin Pharmacol Ther* 1986; **40:** 281-286.
35. Faulds, D., and Heel, R. C. Ganciclovir. A review of its antiviral activity, pharmacokinetic properties and therapeutic efficacy in cytomegalovirus infections. *Drugs* 1990; **39:** 597-638.
36. Buhles, W. C. Clinical safety of intravenous ganciclovir. In: Dekker, M. (ed). *Ganciclovir therapy for cytomegelovirus infection*. New York, 1991, pp 31-69.
37. Morozov, V. A., Noguiez-Hellin, P., Laune, S., Tamboise, E., Salzmann, J. L., and Klatzmann, D. Plasmovirus: replication cycle of a novel nonviral/viral vector for gene transfer. *Cancer Gene Ther* 1997; **4**: 286-293.
38. Kremmer, T., Paulik, E., Boldizsar, M., and Holenyi, I. Application of the fast protein liquid chromatographic system and MonoQ HR 5/5 anion exchanger to the separation of nucleosides. *Journal of Chromatography* 1989; **493:** 45-52.
39. Guettari, N., Loubiere, L., Brisson, E., and Klatzmann, D. Use of herpes simplex virus thymidine kinase to improve the antiviral activity of zidovudine. *Virology* 1997; **235:** 398-405.

## Claims

1. The use of an Equine herpes virus type-4 (EHV4) thymidine kinase polypeptide or a nucleic acid coding therefor, and a nucleoside analog selected from GCV (ganciclovir) and ACV (acyclovir), for the manufacture of a composition for the killing of human proliferative cells, in particular human tumor cells, in vitro, ex vivo or in vivo.

2. The use of an EHV4-TK polypeptide or a nucleic acid coding therefor, and a nucleoside analog selected from GCV (ganciclovir) and ACV (acyclovir), for the manufacture of a composition for the killing of human T lymphocytes in vitro, ex vivo or in vivo.

3. The use of claim 1 or 2, wherein the Equine herpes virus type-4 (EHV4) thymidine kinase polypeptide or nucleic acid and the nucleoside analog are to be administered simultaneously or separately.

4. A pharmaceutical composition for the treatment of human cell proliferative diseases comprising an Equine herpes virus type-4 (EHV4) thymidine kinase polypeptide or a nucleic acid coding therefor and a nucleoside analog selected from GCV (ganciclovir) and ACV (acyclovir), to be administered simultaneously or separately.

5. The pharmaceutical composition of claim 4 wherein the nucleoside analog is GCV.

6. The use of claim 1 or 2, wherein said Equine herpes virus type-4 (EHV4) thymidine kinase polypeptide comprises wild-type EHV4-TK or a biologically active fragment or variant thereof.

7. The use of claim 1 or 2, wherein said nucleic acid encoding an Equine herpes virus type-4 (EHV4) thymidine kinase polypeptide comprises a nucleic acid encoding wild-type EHV4-TK or a nucleic acid which hybridizes therewith and codes for a biologically active EHV4-TK polypeptide.

8. The use according to any one of claims 1, 2, 6 and 7, wherein said nucleic acid is contained in a vector.

9. The use according to claim 8, wherein said vector is a recombinant virus.

10. A method of rendering a human cell sensitive to a nucleoside analog selected from GCV (ganciclovir) and ACV (acyclovir), comprising contacting said human cell in vitro or ex vivo with an Equine herpes virus type-4 (EHV4) thymidine kinase polypeptide or a nucleic acid coding therefor.

11. A method of killing a human cell, comprising contacting said human cell in vitro or ex vivo with an Equine herpes virus type-4 (EHV4) thymidine kinase polypeptide or a nucleic acid coding therefor, and further contacting said cell or its progeny with a nucleoside analog selected from GCV (ganciclovir) and ACV (acyclovir).

## Patentansprüche

1. Die Verwendung eines Thymidinkinase-Polypeptids des Pferdeherpesvirus Typ 4 (EHV4) oder einer dafür codierenden Nukleinsäure und eines aus GCV (Ganciclovir) und ACV (Acyclovir) ausgewählten Nukleosidanalogs zur Herstellung einer Zusammensetzung zur Abtötung menschlicher proliferierender Zellen, insbesondere menschlicher Tumorzellen, in vitro, ex vivo oder in vivo.

2. Die Verwendung eines EHV4-TK-Polypeptids oder einer dafür codierenden Nukleinsäure und eines aus GCV (Ganciclovir) und ACV (Acyclovir) ausgewählten Nukleosidanalogs zur Herstellung einer Zusammensetzung zur Abtötung menschlicher T-Lymphocyten in vitro, ex vivo oder in vivo.

3. Die Verwendung nach Anspruch 1 oder 2 zur gleichzeitigen oder getrennten Verabreichung des Thymidinkinase-Polypeptids des Pferdeherpesvirus Typ 4 (EHV4) oder der Nukleinsäure und des Nukleosidanalogs.

4. Eine pharmazeutische Zusammensetzung zur Behandlung zellproliferativer Erkrankungen des Menschen, umfassend ein Thymidinkinase-Polypeptid des Pferdeherpesvirus Typ 4 (EHV4) oder eine dafür codierende Nukleinsäure und ein aus GCV (Ganciclovir) und ACV (Acyclovir) ausgewähltes Nukleosidanalog zur gleichzeitigen oder getrennten Verabreichung.

5. Die pharmazeutische Zusammensetzung nach Anspruch 4, wobei das Nukleosidanalog GCV ist.

6. Die Verwendung nach Anspruch 1 oder 2, wobei das Thymidinkinase-Polypeptid des Pferdeherpesvirus Typ 4 (EHV4) Wildtyp EHV4-TK oder ein biologisch aktives Fragment oder eine Variante desselben umfasst.

7. Die Verwendung nach Anspruch 1 oder 2, wobei die ein Thymidinkinase-Polypeptid des Pferdeherpesvirus Typ 4 (EHV4) codierende Nukleinsäure eine Wildtyp EHV4-TK codierende Nukleinsäure oder eine damit hybridisierende Nukleinsäure, die ein biologisch aktives EHV4-TK Polypeptid codiert, umfasst.

8. Die Verwendung nach einem der Ansprüche 1, 2, 6 und 7, wobei die Nukleinsäure in einem Vektor enthalten ist.

9. Die Verwendung nach Anspruch 8, wobei der Vektor ein rekombinantes Virus ist.

10. Ein Verfahren zur Sensitivierung einer menschlichen Zelle gegenüber einem Nukleosidanalog ausgewählt aus GCV (Ganciclovir) und ACV (Acyclovir), umfassend das in Kontakt bringen der menschlichen Zelle in vitro oder ex vivo mit einem Thymidinkinase-Polypeptid des Pferdeherpesvirus Typ 4 (EHV4) oder einer dafür codierenden Nukleinsäure.

11. Ein Verfahren zur Abtötung einer menschlichen Zelle, umfassend das in Kontakt bringen der menschlichen Zelle in vitro oder ex vivo mit einem Thymidinkinase-Polypeptid des Pferdeherpesvirus Typ 4 (EHV4) oder einer dafür codierenden Nukleinsäure und weiterhin das in Kontakt bringen der Zelle oder ihrer Nachkommen mit einem Nukleosidanalog ausgewählt aus GCV (Ganciclovir) und ACV (Acyclovir).

## Revendications

1. Utilisation d'un polypeptide de thymidine kinase de l'herpesvirus équin de type 4 (EHV4) ou d'un acide nucléique codant pour celui-ci, et d'un analogue nucléosidique sélectionné parmi le GCV (gancyclovir) et l'ACV (acyclovir), pour la préparation d'une composition destinée à tuer des cellules prolifératives humaines, en particulier des cellules tumorales humaines, in vitro, ex vivo ou in vivo.

2. Utilisation d'un polypeptide de TK-EHV4 ou d'un acide nucléique codant pour celui-ci, et d'un analogue nucléosidique sélectionné parmi le GCV (gancyclovir) et l'ACV (acyclovir), pour la préparation d'une composition destinée à tuer des lymphocytes T humains in vitro, ex vivo ou in vivo.

3. Utilisation selon la revendication 1 ou 2, dans laquelle le polypeptide ou l'acide nucléique de thymidine kinase de l'herpesvirus équin de type 4 (EHV4) et l'analogue nucléosidique sont destinés à être administrés simultanément ou séparément.

4. Composition pharmaceutique pour le traitement de maladies prolifératives des cellules humaines comprenant un polypeptide de thymidine kinase de l'herpesvirus équin de type 4 (EHV4) ou un acide nucléique codant pour celui-ci, et un analogue nucléosidique sélectionné parmi le GCV (gancyclovir) et l'ACV (acyclovir), destinés à être administrés simultanément ou séparément.

5. Composition pharmaceutique selon la revendication 5, dans laquelle l'analogue nucléosidique est le GCV.

6. Utilisation selon la revendication 1 ou 2, dans laquelle ledit polypeptide de thymidine kinase de l'herpesvirus équin de type 4 (EHV4) comprend la TK-EHV4 sauvage ou un fragment ou variant biologiquement actif de celle-ci.

7. Utilisation selon la revendication 1 ou 2, dans laquelle ledit acide nucléique codant le polypeptide de thymidine kinase de l'herpesvirus équin de type 4 (EHV4) comprend un acide nucléique codant la TK-EHV4 sauvage ou un acide nucléique qui s'hybride avec celui-ci et code un polypeptide TK-EHV4 biologiquement actif.

8. Utilisation selon l'une quelconque des revendications 1, 2, 6 et 7, dans laquelle ledit acide nucléique est contenu dans un vecteur.

9. Utilisation selon la revendication 8, dans laquelle ledit vecteur est un virus recombinant.

10. Méthode pour rendre une cellule humaine sensible à un analogue nucléosidique sélectionné parmi le GCV (gancyclovir) et l'ACV (acyclovir), comprenant la mise en contact de ladite cellule humaine in vitro ou ex vivo avec un polypeptide de thymidine kinase de l'herpesvirus équin de type 4 (EHV4) ou un acide nucléique codant pour celui-ci.

11. Méthode pour tuer une cellule humaine, comprenant la mise en contact de ladite cellule humaine in vitro ou ex vivo avec un polypeptide de thymidine kinase de l'herpesvirus équin de type 4 (EHV4) ou un acide nucléique codant pour celui-ci, et en outre la mise en contact de ladite cellule ou sa descendance avec un analogue nucléosidique sélectionné parmi le GCV (gancyclovir) et l'ACV (acyclovir).
